# EUROPEAN PATENT APPLICATION

(11) **EP 3 174 069 A1**
(43) Date of publication of application: **31.05.2017**
(21) Application number: 15196840.1
(22) Date of filing: 27.11.2015
(51) Int. Cl.: G21K 1/10

(54) **DEGRADER COMPRISING BORON CARBIDE**

(71) Applicant: Paul Scherrer Institut, 5232 Villigen (CH)
(72) Inventor: Schippers, Jacobus Maarten, 5236 Remigen (CH); Gerbershagen, Alexander, 5200 Brugg (CH)
(74) Representative: Fischer, Michael

(57) **Abstract**

It is the objective of the present invention to provide a degrader that has excellent degrading capabilities with, for the same energy loss in the degrader, a lower emittance increase as currently used materials, without generating a strong neutron flux and without having severe toxic characteristics.

This objective is achieved according to the present invention by a degrader (2) for use in the field of the application of a particle beam (6), comprising degrading active material wherein the degrading active material comprises Boron Carbide B₄C.

This degrader has an amount of multiple scattering that is lower than in graphite for the same energy loss. The use of B₄C increases the transmission by at typically 35% for the beam degradation to low energies, which is a significant and useful amount of beam intensity increase in particle therapy. The B₄C-material does not become more radio-active than graphite, so that there will be no additional problems at service activities. Further, B₄C as degrading active material does not have severe toxic properties.

## Description

The present invention relates to a degrader, preferably used in particle radiation therapy facilities.

When slowing down (="degrading") protons or ions (particles) from a particle accelerator, such as for example a cyclotron, to an energy compatible with the range of the particles in tissue, this is done by sending the beam through a degrader. A degrader is an amount of material (a block or several layers) with a low atomic number (Z), such as graphite. The degrader, or a part of it, is inserted in the trajectory of the particle beam in order to reduce the energy of the particles. The total thickness of the amount of this material determines the energy of the out-going particles. However, the particles crossing such material are also scattered by the nuclei in the material. Due to this so-called multiple scattering process the out-going beam obtains an increase in diameter as well as an increase in angular spread (divergence). In order to prevent unwanted beam losses in the beam transport system following the degrader, usually a set of collimators is mounted immediately behind the degrader. When degrading to low energies the scattering increases. At these collimators the losses thus increase, which yields a decrease of the transmission, which is the fraction of the beam intensity from the accelerator, that reaches the patient. In proton therapy this causes an undesirable increase of treatment time.

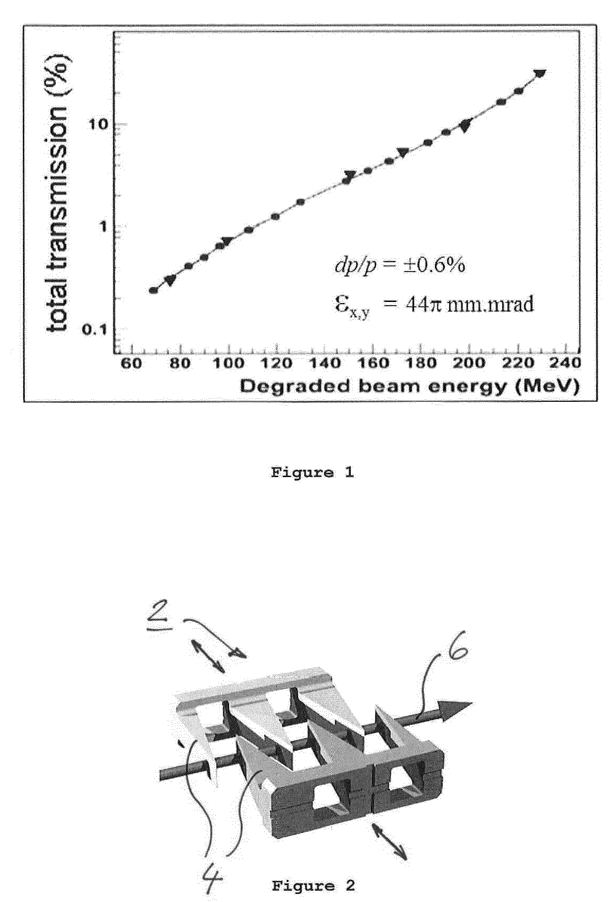

At Paul Scherrer Institute (PSI), CH-5232 Villigen PSI, the proton therapy facility PROSCAN consists of a 250 MeV superconducting cyclotron COMET and a beam transport system that guide the beam to four treatment areas, Gantry 1, Gantry 2, Gantry 3 and OPTIS. The energy required for the patient treatment is in the range between 70 MeV and 230 MeV. Energy modulation is performed via a graphite degrader (Figure 2), that is inserted into the beam trajectory, reducing the beam energy from 250 MeV to a value specified by the treatment planning.

Low energies are used in the treatments in the gantries for dose deposition in the (parts of the) tumors that are located at a shallow position below the skin. The lowest beam energy of 70 MeV requires the maximum energy degradation in PROSCAN and beam transmission is most challenging (see transmission curve in Figure 1). This energy is also used for treatments of eye melanoma at OPTIS. Active participation of the patients is required for eye treatments. The irradiation time should therefore be as short as possible and, hence, a reasonably high beam intensity is important at the patient.

Unfortunately, the multiple scattering process in an energy degrader increases the beam size, divergence and energy spread beyond the beamline and gantry acceptance. It is unavoidable to use one or more collimator systems and an energy selection system to cut these quantities to those that fit in the acceptance of the following beam transport system, to prevent unwanted beam losses in the beam transport system. This limited acceptance depends on the energy, the geometrical layout of the beam transport system and on the setting of the magnets in this system. At PSI a typical beam transmission is on the order of 0.2% for beams with an energy of 70 MeV.

Typically, for lower energies the intensity loss can be compensated by an increase of beam current at the cyclotron exit. The maximum beam current provided by the cyclotron is, however, limited. Hence, it is useful to examine the possibilities of reducing the emittance increase in the degrader that leads to the beam loss in the subsequent beam transport system.

The usual choice for degrader material is graphite, so a material with a low atomic number Z, to limit the multiple scattering amplitude. Beryllium is also a degrader material with a low Z, thus also causing a low divergence increase and it has been used as degrader material at some places. However, it has a disadvantage of creating a strong neutron flux during the degrading of high energy protons. Furthermore, Beryllium is known to have some toxic characteristics. Plastics are also used, but these suffer from geometry changes due to the radiation damage and heat deposition in the material.

It is therefore the objective of the present invention to provide a degrader that has excellent degrading capabilities without generating neutron flux and not having toxic properties.

This objective is achieved according to the present invention by a degrader comprising degrading active material wherein the degrading active material comprises Boron Carbide B₄C.

This degrader evokes an amount of multiple scattering that is lower than in graphite for the same energy loss. At PSI it has been found that the use of B₄C increases the transmission by at least 35% for the beam degradation to low energies, which is a significant and useful amount of beam intensity increase in particle therapy. The B₄C-material does not become more radioactive than graphite, so that there will be no additional problems at service activities. Further, B₄C as degrading active material does not have any toxic properties.

Suitably, the degrading active material can be assembled as plates or as wedges.

Suitably, the degrading active material can be mounted on one or more actuators that can bring the degrader or a part of the degrader in a position where it is crossed by the particle beam.

Preferred embodiments of the present invention are hereinafter described in more detail with reference to the attached drawings which depict in:
- Figure 1: Transmission of a proton current through a degrader, emittance collimators and energy selection section for a beam with an initial energy of 250 MeV, as measured at PSI;
- Figure 2: a depiction of degrader wedges in the beam trajectory as currently in use at PSI; and

Test measurements using boron carbide (B₄C) as degrader material are discussed in the following in comparison with the conventional graphite, which is currently used in many proton therapy degraders. B₄C is a material of lower average atomic weight and higher density than graphite. Calculations predict that, compared with graphite, the use of B₄C results in a lower emittance behind the degrader. Downstream of the acceptance defining collimation system at the entrance of the following beam lines, a higher beam transmission occurs, especially at low beam energies. This is of great interest in particle therapy applications as it allows either a reduction of the beam intensity extracted from the cyclotron or a reduction of the treatment time.

The results of experiments carried out at the PROSCAN facility at the Paul Scherrer Institute are discussed hereinafter. The simulations of a B₄C -degrader have predicted an increase in the beam transmission of approx. 31% compared to graphite, for beam degradation from 250 to 84 MeV. The experiment carried out with a B₄C block reducing the energy to 84 MeV yielded a transmission improvement of 37% compared with the carbon degrader set to that energy.

In the experiment a B₄C block has been used, having a length of 150 mm and transverse dimensions 24 mm x 24 mm, mounted in an aluminum frame with open ends at beam entrance and exit. The block has been installed in the degrader box at the position of the carbon wedges and aligned in order to provide the same position at the beam exit. The conditions of the transmission measurement with the B₄C degrader were similar to those with the graphite degrader.

Fig. 2 shows the graphite degrader 2 having wedges 4 which can be driven into or out of the particle beam 6 in a direction perpendicular to the direction of the particle beam 6. Other B₄C degrader might have a similar form in order to be capable of changing the beam energy dynamically. However, this is not the only layout: there are other options possible, e.g. with only one long wedge and/or rotational wheel or something else.

The beam current has been measured after the cyclotron and after the energy selection system for both the B₄C block and the graphite wedge degrader for a beam energy of 84 MeV. The transmission, defined as the ratio of these beam currents, was 0.59 % for the B₄C block and 0.43 % for the graphite degrader. Hence, it was found that the measured transmission is 37.2% higher for the B₄C block compared to the graphite degrader for the same energy.

Measurements have successfully been performed to compare the experimental behavior with the calculated performance of B₄C as potential degrader material. Both simulation and measurement yield an improvement of the transmission at 84 MeV in the order of 35% using a B₄C degrader compared to the currently used graphite degrader. Further a 30 % lower dose rate at the B₄C degrader is expected compared with the graphite degrader. Thus, the level of B₄C activity after the irradiation is considered as acceptable and uncritical with respect to machine maintenance.

## Claims

1. A degrader (2) for use in the field of the application of a particle beam (6), comprising degrading active material wherein the degrading active material comprises Boron Carbide (B₄C).

2. The degrader according to claim 1 wherein the degrading active material is mounted on one or more actuators that can bring the degrader or a part of the degrader in a position where it is crossed by the particle beam.

3. The degrader (2) according to claim 1 wherein the degrading active material is assembled as one element (block) or comprises a number of elements, such as wedges (4) and/or plates.
